# EUROPÄISCHE PATENTSCHRIFT

(11) **EP 4 112 284 B1**
(45) Veröffentlichungstag und Bekanntmachung des Hinweises auf die Patenterteilung: **30.07.2025**
(21) Anmeldenummer: 22180094.9
(22) Anmeldetag: 21.06.2022
(51) Int. Cl.: B29C 71/02, B29C 71/04, A61L 27/16, A61L 27/50, A61L 27/54, A61L 27/56, B29K 23/00

(54) **VERFAHREN UND SYSTEM ZUR HERSTELLUNG VON FORMKÖRPERN AUF BASIS VON VERNETZTEM UHMWPE SOWIE FORMKÖRPER AUF BASIS VON VERNETZTEM UHMWPE**
MOULDINGS BASED ON CROSSLINKED UHMWPE AND METHOD AND SYSTEM FOR PRODUCING MOULDINGS BASED ON CROSSLINKED UHMWPE
PROCÉDÉ ET SYSTÈME DE FABRICATION DE CORPS MOULÉS À BASE D'UHMWPE RÉTICULÉ, AINSI QUE CORPS MOULÉ À BASE D'UHMWPE RÉTICULÉ

(30) Priorität: 02.07.2021 DE 102021117155
(43) Veröffentlichungstag der Anmeldung: 04.01.2023
(73) Patentinhaber: Aesculap AG, 78532 Tuttlingen (DE)
(72) Erfinder: Fabritius, Martin, 78532 Tuttlingen (DE)
(74) Vertreter: Hoeger, Stellrecht & Partner Patentanwälte mbB

(56) Entgegenhaltungen:
- US-A1- 2012 016 051
- US-A1- 2015 151 866
- US-B1- 6 355 215

## Beschreibung

Die vorliegende Erfindung betrifft ein Verfahren zur Herstellung von einem oder mehreren Formkörpern auf Basis von vernetztem UHMWPE.

Die Erfindung betrifft ferner einen Formkörper auf Basis von vernetztem UHMWPE, welcher gemäß diesem Verfahren hergestellt ist.

Des Weiteren betrifft die Erfindung ein System zur Herstellung von einem oder mehreren Formkörpern auf Basis von vernetztem UHMWPE mit einer Aufnahmevorrichtung für einen oder mehrere Formkörper aus verdichtetem UHMWPE.

Es sind seit längerem Gelenkendoprothesen bekannt, die Formkörper aus ultrahochmolekularem Polyethylen (UHMWPE) als Gleitflächenelemente umfassen. Dabei wirkt das Gleitflächenelement aus UHMWPE in der Regel mit einem korrespondierenden Element aus einem metallischen Material zusammen, wobei sich mit dieser Materialkombination ein niedriger Reibungskoeffizient ergibt. Bei einer Hüftgelenkprothese ist das Gleitflächenelement aus UHMWPE typischerweise als Inlay des Hüftkopfimplantats ausgebildet, während es bei einer Kniegelenkprothese typischerweise an der Tibiakomponente festgelegt ist und auch als Meniskuselement bezeichnet wird. Auch bei einer Kniescheibenprothese (Patellaprothese) kann eine Gleitfläche aus UHMWPE eingesetzt werden.

Das Gleitflächenelement muss hinsichtlich seiner mechanischen Eigenschaften, Verschleißfestigkeit und Alterungsresistenz bestimmte Voraussetzungen erfüllen, wobei die hierfür erforderlichen Materialeigenschaften des UHMWPE zum Teil gegenläufig sind. Zur Erhöhung der Verschleißfestigkeit kann UHMWPE mit ionisierender Strahlung vernetzt werden, wie z.B. in der EP 0 995 450 A1 beschrieben. Gleichzeitig reduziert die Vernetzung jedoch die Duktilität des Materials, wodurch sich das Risiko einer strukturellen Materialermüdung, einer Delamination und gegebenenfalls eines Versagens des Gleitflächenelements erhöht.

Da bei der Strahlungsvernetzung von UHMWPE freie Radikale entstehen und teilweise in dem Material verbleiben, führt dies tendenziell zu einer schlechteren Alterungsresistenz aufgrund von oxidativen Prozessen. Diesem Problem kann mindestens teilweise durch den Zusatz von Antioxidantien wie zum Beispiel α-Tocopherol (Vitamin E) entgegengewirkt werden, wobei das Antioxidans entweder bereits vor der Herstellung eines Formkörpers dem UHMWPE zugesetzt wird, oder erst nach dem Vernetzen durch Diffusion in den Formkörper eingebracht wird (siehe zum Beispiel die EP 3 111 895 A1). Vor dem Hintergrund, dass zunehmend jüngere Patienten mit Gelenkendoprothesen versorgt werden, und dass gleichzeitig die Lebenserwartung steigt und die Patienten auch in höherem Alter aktiv bleiben, kommt der Alterungsresistenz von UHMWPE in Gelenkendoprothesen eine steigende Bedeutung zu.

Einen relevanten Einfluss auf die Eigenschaften von Formkörpern aus vernetztem UHMWPE haben unter anderem auch die Art der ionisierenden Strahlung sowie die Strahlendosis, mit denen die Bestrahlung durchgeführt wird. Die Verwendung von Gammastrahlung hat zum Beispiel gegenüber Betastrahlung den Vorteil, dass die eingesetzte Strahlendosis besser kontrollierbar ist und damit das Ergebnis der Strahlungsvernetzung eine höhere Reproduzierbarkeit aufweist. Aufgrund der wesentlich geringeren Strahlungsintensität bei Gammastrahlung liegen hier die Bestrahlungszeiten typischerweise im Bereich von einigen Stunden, im Gegensatz zu einigen Sekunden oder Minuten bei Betastrahlung. Als Alternative zu Gammastrahlung kann auch mit Röntgenstrahlung vernetzt werden, wie z.B. in der EP 3 510 084 B1 beschrieben.

Im Hinblick auf eine möglichst hohe Verschleißfestigkeit und Alterungsresistenz von Formkörpern aus vernetztem UHMWPE ist es generell wünschenswert, einen hohen Vernetzungsgrad mit einer möglichst geringen Strahlendosis zu erreichen. Hierzu ist es vorteilhaft, wenn die Bestrahlung bei einer erhöhten Temperatur des Formkörpers durchgeführt wird, insbesondere bei einer Temperatur im Bereich der Schmelztemperatur des UHMWPE oder darüber, da dies zu einer höheren Beweglichkeit der Polymerketten und Effektivität der Vernetzungsreaktion führt. Da eine direkte Erwärmung der Formkörper in der Bestrahlungsvorrichtung schwierig zu realisieren ist, wird gemäß dem Stand der Technik überwiegend so verfahren, dass die zu bestrahlenden Formkörper zunächst auf eine bestimmte Temperatur vorgewärmt und anschließend bestrahlt werden. Dabei lässt sich jedoch nicht vermeiden, dass die Formkörper bereits während der Bestrahlung wieder abkühlen, wobei das Ausmaß dieser Abkühlung nicht genau kontrollierbar ist (zum Beispiel während der Überführung von einem Ofen in die Bestrahlungsvorrichtung). Dies hat einen negativen Einfluss auf die Reproduzierbarkeit der Strahlungsvernetzung. In der DE 10 2013 113 781 A1 wird vorgeschlagen, die Formkörper während der Bestrahlung in einer thermisch isolierenden Verpackungseinheit anzuordnen, um das Abkühlen während der Bestrahlung zu begrenzen. Das Problem der mangelnden Reproduzierbarkeit aufgrund der Temperaturänderung der Formkörper lässt sich dadurch jedoch allenfalls teilweise lösen.

US 2012/016051 A1 und US 2015/151866 A1 offenbaren ein Verfahren zur Herstellung von einem oder mehreren Formkörpern auf Basis von vernetztem UHMWPE.

Es ist die Aufgabe der vorliegenden Erfindung, ein Verfahren zur Herstellung von einem oder mehreren Formkörpern auf Basis von vernetztem UHMWPE vorzuschlagen, welches im Hinblick auf den Vernetzungsgrad der hergestellten Formkörper möglichst effizient und reproduzierbar ist.

Diese Aufgabe wird erfindungsgemäß dadurch gelöst, dass das Verfahren folgende Schritte umfasst:
- Verdichten von UHMWPE in Pulverform zu einem oder mehreren Formkörpern;
- Einbringen des oder der Formkörper in einen Aufnahmeraum einer Aufnahmevorrichtung, wobei die Aufnahmevorrichtung ein Heizmittel zum Erwärmen des oder der in dem Aufnahmeraum befindlichen Formkörper umfasst;
- Bestrahlen des oder der Formkörper in der Aufnahmevorrichtung mit Gamma- oder Röntgenstrahlung, um das UHMWPE zu vernetzen; und
- Entnehmen des oder der Formkörper aus dem Aufnahmeraum,
wobei die Aufnahmevorrichtung dafür ausgelegt ist, einen vorgegebenen Temperaturverlauf des oder der Formkörper vor, während und nach dem Bestrahlen zu steuern.

Die wesentliche Neuerung des erfindungsgemäßen Verfahrens besteht somit darin, dass der oder die Formkörper während des Bestrahlens innerhalb einer Aufnahmevorrichtung angeordnet sind, die eine aktive Erwärmung und eine Steuerung des Temperaturverlaufs des oder der Formkörper ermöglichen. Auf diese Weise kann insbesondere die Temperatur, bei der die Bestrahlung erfolgt, sehr genau vorgegeben und eingestellt werden, so dass die Vernetzung des UHMWPE unter definierten und reproduzierbaren Bedingungen erfolgt.

Die Aufnahmevorrichtung ist im Sinne der vorliegenden Erfindung eine separate Einheit, die von der Bestrahlungsvorrichtung unabhängig ist. Somit verzichtet die Erfindung auf eine technisch aufwendige und mit hohen Kosten verbundene Modifikation der für das Verfahren eingesetzten Bestrahlungsvorrichtung. Stattdessen ist die verwendete Aufnahmevorrichtung mit herkömmlichen Bestrahlungsvorrichtungen verwendbar und wird im Zuge des Verfahrens in deren Bestrahlungsraum eingeführt, wobei lediglich beachtet werden muss, dass die Aufnahmevorrichtung entsprechend dimensioniert ist, und dass ihre Wandung aus einem für Gamma- und Röntgenstrahlung durchlässigen Material besteht.

Es ist bevorzugt, wenn der oder die Formkörper nach dem Einbringen in den Aufnahmeraum und vor dem Bestrahlen durch das Heizelement auf eine Zieltemperatur erwärmt werden. Die Zieltemperatur kann in Abhängigkeit von verschiedenen Faktoren gewählt und vorgegeben werden, wobei es besonders vorteilhaft ist, bei der Wahl der Zieltemperatur die vorgesehene Strahlendosis und/oder Bestrahlungszeit zu berücksichtigen. Durch die Möglichkeit, diese wesentlichen Verfahrensparameter genau aufeinander abzustimmen, erlaubt das erfindungsgemäße Verfahren eine besonders effiziente Vernetzung des oder der Formkörper.

Die Zieltemperatur liegt günstigerweise im Bereich der Schmelztemperatur des UHMWPE (etwa 137 °C) oder darüber. Durch die höhere Beweglichkeit der Polymerketten in diesem Temperaturbereich ist die Strahlungsvernetzung generell effektiver als bei einer Temperatur unterhalb des Schmelzbereichs.

Bevorzugt beträgt die Zieltemperatur bei dem erfindungsgemäßen Verfahren von 135 bis 150 °C, weiter bevorzugt von 137 bis 145 °C.

Bei einer bevorzugten Ausführungsform des Verfahrens werden der oder die Formkörper während dem Bestrahlen im Wesentlichen konstant bei der Zieltemperatur gehalten. Alternativ kann auch vorgesehen sein, dass die Temperatur des oder der Formkörper während dem Bestrahlen geändert wird.

Nach dem Bestrahlen und vor dem Entnehmen aus dem Aufnahmeraum werden der oder die Formkörper vorzugsweise abkühlen gelassen. Dabei kann optional die Abkühlzeit verlängert werden, indem das Heizmittel auch während dieser Phase in einem gewissen Umfang aktiviert wird.

Im Sinne des erfindungsgemäßen Verfahrens wird somit nicht nur eine konstante Zieltemperatur während dem Bestrahlen vorgegeben und gesteuert, sondern es ist prinzipiell möglich, den gesamten Temperaturverlauf des oder der Formkörper vom Einbringen in den Aufnahmeraum (typischerweise bei Umgebungstemperatur) bis zum Entnehmen des oder der Formkörper aus dem Aufnahmeraum vorzugeben und zu regeln. Zu diesem Zweck umfasst die Aufnahmevorrichtung günstigerweise ein Steuermittel und einen oder mehrere Temperatursensoren.

Als Heizmittel kann die Aufnahmevorrichtung grundsätzlich jede Art von Einrichtung umfassen, die ein Erwärmen des oder der in dem Aufnahmeraum befindlichen Formkörper ermöglicht. Insbesondere kann das Heizmittel ein Heißluftgebläse, einen Infrarotstrahler oder ein elektrisches Heizelement umfassen.

Die Aufnahmevorrichtung umfasst bevorzugt eine von der Umgebung unabhängige Energieversorgung, insbesondere einen Akkumulator. Der Akkumulator muss ausreichend dimensioniert sein, um den oder die Formkörper auf die Zieltemperatur zu erwärmen und die entsprechende Temperatur zumindest während der Zeitdauer der Bestrahlung halten zu können. Alternativ kann auch vorgesehen sein, dass die Aufnahmevorrichtung an eine externe Stromversorgung angeschlossen wird.

Die Aufnahmevorrichtung weist bevorzugt eine äußere Wandung aus einem thermisch isolierenden Material auf. Dadurch kann der Energiebedarf für das Erwärmen und Halten der Temperatur reduziert werden. Das Material der äußeren Wandung der Aufnahmevorrichtung muss zudem, wie bereits oben erwähnt, für Gamma- und Röntgenstrahlung durchlässig sein.

Das Bestrahlen des oder der Formkörper wird typischerweise mit einer Strahlendosis von 25 bis 45 kGy durchgeführt, bevorzugt von 27 bis 33 kGy, weiter bevorzugt von ca. 30 kGy. Es hat sich gezeigt, dass mit einer Strahlendosis in dieser Größenordnung sehr günstige Eigenschaften des vernetzten Formkörpers erhalten werden können, insbesondere im Hinblick auf Verschleißfestigkeit und Duktilität.

Das Bestrahlen des oder der Formkörper mit Gamma- oder Röntgenstrahlung wird vorzugsweise über einen Zeitraum von 2 bis 6 h durchgeführt, bevorzugt von 3 bis 4 h. Bei einer bevorzugten Strahlendosis von 30 kGy entspricht dies einer Dosisrate im Bereich von 5 bis 15 kGy/h.

Bei einer bevorzugten Ausführungsform des erfindungsgemäßen Verfahrens wird das UHMWPE in Pulverform vor dem Verdichten mit einem Antioxidans versetzt. Wie bereits eingangs erwähnt, kann durch ein Antioxidans die Alterungsresistenz von strahlungsvernetztem UHMWPE verbessert werden, indem das Antioxidans mit den nach der Bestrahlung verbleibenden freien radikalen reagiert.

Als Antioxidans, mit dem das UHMWPE versetzt wird, können verschiedene Antioxidationsmittel eingesetzt werden, die für eine Verwendung auch im medizinischen Bereich zugelassen sind. Bevorzugt ist das Antioxidans ausgewählt aus Tocopherolen, Tocotrienolen, Ascorbinsäure, polyphenolischen Antioxidantien wie z. B. Flavonoiden, Butylhydroxytoluol (BTH) und Butylhydroxyanisol (BTA).

Bei einer bevorzugten Ausführungsform der Erfindung ist das Antioxidans α-Tocopherol. Dieses wird auch als Vitamin E bezeichnet, wobei der Begriff Vitamin E in einem weiteren Sinne alle Tocopherole, Tocotrienole und weitere fettlösliche Antioxidantien umfasst.

Der Anteil des Antioxidans, mit dem das UHMWPE versetzt wird, beträgt günstigerweise von 0,05 bis 0,15 Gew.%, bezogen auf das UHMWPE, bevorzugt von 0,09 bis 0,11 Gew.%.

Das im Rahmen der Erfindung eingesetzte UHMWPE weist typischerweise ein Molekulargewicht im Bereich von 5·10⁶ bis 10⁷ g/mol auf (ermittelt aus der intrinsischen Viskosität) und eine Dichte im Bereich von 0,92 bis 0,95 g/cm³. Ein geeignetes UHMWPE in Pulverform ist z.B. von der Ticona GmbH unter der Bezeichnung GUR 1020 erhältlich, bzw. als Mischung mit 0,1 Gew.% α-Tocopherol als Antioxidans versetzt unter der Bezeichnung GUR 1020-E.

Das Verdichten des UHMWPE zu einem Formkörper erfolgt typischerweise mittels Formpressen oder RAM-Extrusion, bevorzugt bis zu einer Dichte des Formkörpers von 0,92 g/cm³ oder mehr, d.h. im Wesentlichen bis zum Erreichen der theoretischen Dichte des UHMWPE.

Aus einem verdichteten Formkörper aus UHMWPE können durch materialabtragende Bearbeitung, insbesondere mittels Fräsen, eines oder mehrere der eigentlichen Endprodukte hergestellt werden. Im Rahmen der vorliegenden Erfindung handelt es sich bei diesen Endprodukten insbesondere um ein Gleitflächenelement für eine Gelenkendoprothese, oder auch um andere medizinische Implantate aus vernetztem UHMWPE.

Die materialabtragende Bearbeitung des Formkörpers kann innerhalb des erfindungsgemäßen Verfahrens erfolgen, und zwar nach dem Verdichten des UHMWPE und vor dem Einbringen des oder der Formkörper in die Aufnahmevorrichtung. In diesem Fall entsprechen die Formkörper, die mit Gamma- oder Röntgenstrahlung bestrahlt werden, in ihrer räumlichen Form bereits den herzustellenden Gleitflächenelementen oder sonstigen medizinischen Implantaten.

Alternativ kann die materialabtragende Bearbeitung des oder der Formkörper auch nach dem Bestrahlen und Entnehmen aus dem Aufnahmeraum erfolgen, d.h. im Anschluss an das erfindungsgemäße Verfahren. In diesem Fall handelt es sich bei den erfindungsgemäß hergestellten Formkörpern um Rohlinge aus vernetztem UHMWPE, z.B. in Form von Platten oder Zylindern.

Gegenstand der vorliegenden Erfindung ist ferner ein Formkörper auf Basis von vernetztem UHMWPE, der nach dem erfindungsgemäßen Verfahren hergestellt ist.

Die besonderen Vorteile und bevorzugten Ausführungsformen des erfindungsgemäßen Formkörpers wurden bereits im Zusammenhang mit dem erfindungsgemäßen Verfahren erläutert.

Der erfindungsgemäße Formkörper ist vorzugsweise ein Gleitflächenelement für eine Gelenkendoprothese, insbesondere für eine Hüftgelenkendoprothese oder eine Kniegelenkendoprothese. Gemäß einer weiteren Ausführungsform ist der erfindungsgemäße Formkörper ein Rohling zur Herstellung eines derartigen Gleitflächenelements durch materialabtragende Bearbeitung.

Ein weiterer Gegenstand der vorliegenden Erfindung ist ein System zur Herstellung von einem oder mehreren Formkörpern auf Basis von vernetztem UHMWPE mit einer Aufnahmevorrichtung. Die Aufnahmevorrichtung umfasst einen Aufnahmeraum zur Aufnahme von einem oder mehreren Formkörpern aus verdichtetem UHMWPE und ein Heizmittel zum Erwärmen des oder der in dem Aufnahmeraum befindlichen Formkörper, und mit einer Bestrahlungsvorrichtung zum Bestrahlen des oder der Formkörper (16) in der Aufnahmevorrichtung (10; 30; 40) mit Gamma- oder Röntgenstrahlung, um das UHMWPE zu vernetzen, wobei die Aufnahmevorrichtung dafür ausgelegt ist, einen vorgegebenen Temperaturverlauf des oder der Formkörper vor, während und nach dem Bestrahlen zu steuern.

Wesentliche Vorteile und bevorzugte Ausführungsformen des erfindungsgemäßen Systems wurden ebenfalls bereits im Zusammenhang mit dem erfindungsgemäßen Verfahren beschrieben. Die in diesem Zusammenhang erläuterten Merkmale der Aufnahmevorrichtung können im Rahmen der Erfindung jeweils einzeln oder in beliebiger Kombination verwirklicht sein, sofern sich aus dem jeweiligen Zusammenhang nichts anderes ergibt.

Die vorliegende Offenbarung betrifft ferner die Verwendung des erfindungsgemäßen Systems in einem Verfahren zur Herstellung von einem oder mehreren Formkörpern auf Basis von vernetztem UHMWPE, insbesondere in dem erfindungsgemäßen Verfahren.

Die nachfolgenden Ausführungsbeispiele dienen der näheren Erläuterung der Erfindung, ohne diese in irgendeiner Weise zu beschränken.

Es zeigen im Einzelnen:
- Figur 1:: schematische Darstellung eines ersten Ausführungsbeispiels eines erfindungsgemäßen Systems mit einer Aufnahmevorrichtung;
- Figur 2:: schematische Darstellung eines zweiten Ausführungsbeispiels eines erfindungsgemäßen Systems mit einer Aufnahmevorrichtung;
- Figur 3:: schematische Darstellung eines dritten Ausführungsbeispiels eines erfindungsgemäßen Systems mit einer Aufnahmevorrichtung; und
- Figur 4:: Diagramm zur Darstellung eines vorgegebenen Temperaturverlaufs gemäß einem Ausführungsbeispiel des erfindungsgemäßen Verfahrens.

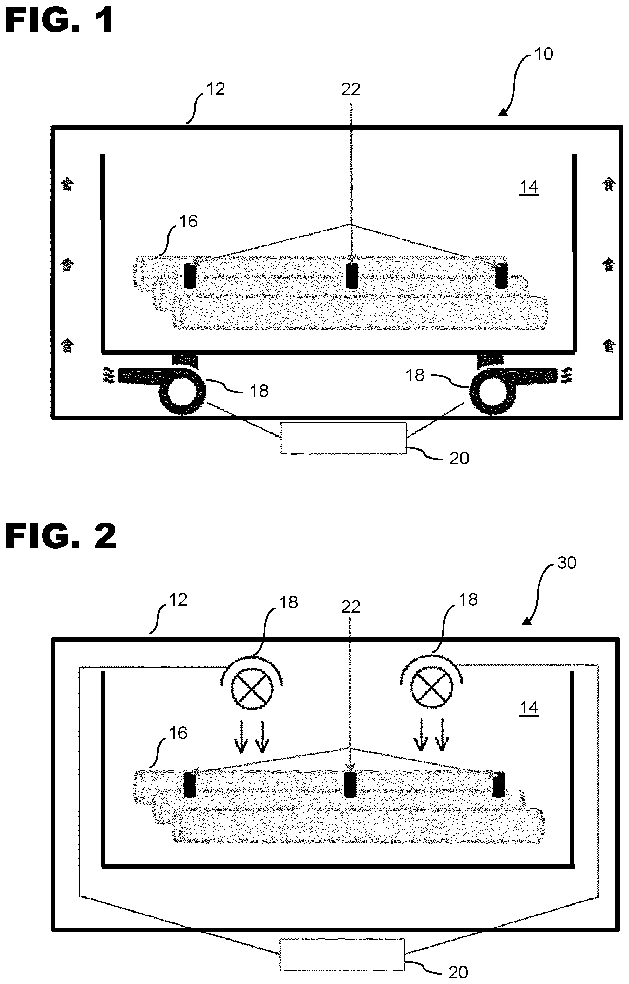

Die Figur 1 zeigt schematisch ein erstes Ausführungsbeispiel eines erfindungsgemäßen Systems mit einer Aufnahmevorrichtung 10. Die Aufnahmevorrichtung 10 weist eine äußere Wandung 12 auf, die aus einem für Gamma- und Röntgenstrahlung durchlässigen und bevorzugt thermisch isolierenden Material besteht. Innerhalb der Aufnahmevorrichtung 10 befindet sich ein Aufnahmeraum 14, in den ein oder mehrere Formkörper 16 durch eine verschließbare Öffnung in der äußeren Wandung 12 (in der Figur nicht dargestellt) eingebracht werden.

Innerhalb der Aufnahmevorrichtung 10 sind ferner zwei Heißluftgebläse als Heizmittel 18 angeordnet, die durch einen Akkumulator 20 mit elektrischer Energie versorgt werden. Der Akkumulator 20 ist in diesem Ausführungsbeispiel außerhalb der Wandung 12 der Aufnahmevorrichtung 10 angeordnet, alternativ ist aber auch eine Anordnung innerhalb der Wandung 12 möglich.

Mittels der Heißluftgebläse 18 können die in dem Aufnahmeraum 14 angeordneten Formkörper 16 auf eine vorgegebene Temperatur erwärmt und bei dieser Temperatur gehalten werden. Bei der Durchführung des erfindungsgemäßen Verfahrens mit der Aufnahmevorrichtung 10 kann z.B. vorgesehen sein, dass die Formkörper 16 vor Beginn der Bestrahlung auf die Zieltemperatur erwärmt werden, während der Zeitdauer der Bestrahlung bei dieser Zieltemperatur gehalten werden, und nach dem Ende der Bestrahlung innerhalb der Aufnahmevorrichtung abkühlen gelassen werden.

Zur Steuerung oder Regelung eines derartigen Temperaturverlaufs umfasst die Aufnahmevorrichtung 10 ferner drei Temperatursensoren 22 in unmittelbarer Nähe der Formkörper 16 sowie ein in der Figur nicht dargestelltes Steuermittel.

Die Figur 2 zeigt schematisch ein zweites Ausführungsbeispiel eines erfindungsgemäßen Systems mit einer Aufnahmevorrichtung 30. Diese entspricht weitgehend der Aufnahmevorrichtung 10 des ersten Ausführungsbeispiels, mit dem Unterschied, dass in diesem Fall zwei Infrarotstrahler als Heizmittel 18 vorgesehen sind. Die Infrarotstrahler 18 sind in räumlicher Nähe der Formkörper 16 so angeordnet, dass deren direkte Erwärmung möglich ist.

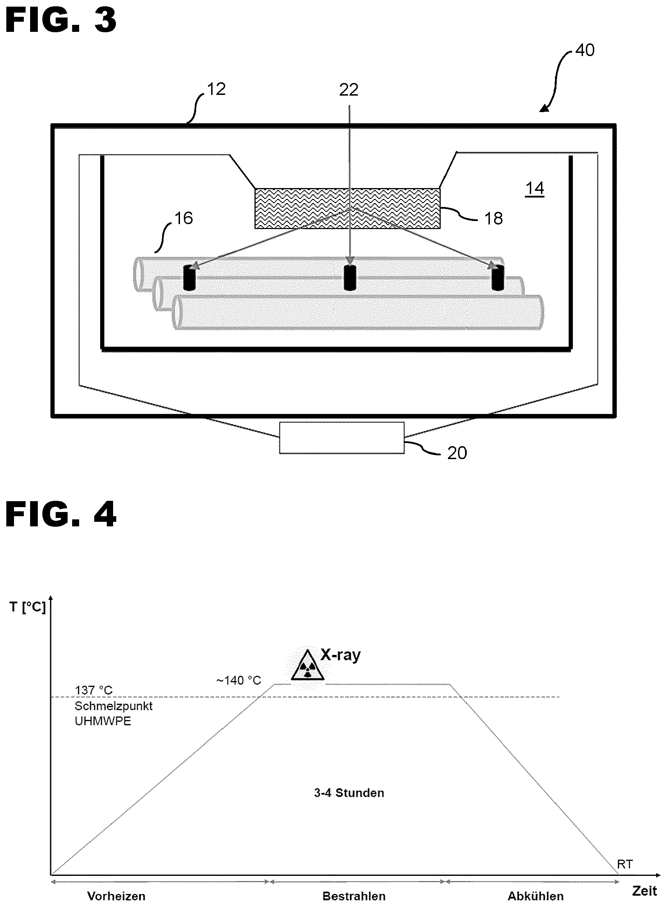

Die Figur 3 zeigt eine schematische Darstellung eines dritten Ausführungsbeispiels eines erfindungsgemäßen Systems mit einer Aufnahmevorrichtung 40. Auch die Aufnahmevorrichtung 40 entspricht im Wesentlichen den Aufnahmevorrichtungen der ersten beiden Ausführungsbeispiele, jedoch mit dem Unterschied, dass als Heizmittel 18 in diesem Fall ein elektrisches Heizelement vorgesehen ist. Das Heizelement 18 kann z.B. in Form einer Heizdecke ausgebildet sein, die in unmittelbarer Nähe der Formkörper 16 angeordnet ist.

Die Aufnahmevorrichtungen 10, 30 und 40 gemäß den vorstehend beschriebenen Ausführungsbeispielen können insbesondere zur Durchführung des erfindungsgemäßen Verfahrens zur Herstellung von einem oder mehreren Formkörpern auf Basis von vernetztem UHMWPE verwendet werden. Hierbei wird UHMWPE in Pulverform, bevorzugt unter Zusatz eines Antioxidans, zu einem oder mehreren Formkörpern 16 verdichtet. Diese werden in den Aufnahmeraum 16 der Aufnahmevorrichtung 10, 30 oder 40 eingebracht und durch die Heizmittel 18 erwärmt, bevorzugt bis zu einer vorgegebenen Zieltemperatur.

Die Aufnahmevorrichtung 10, 30 oder 40 mit den Formkörpern 16 wird in eine Bestrahlungsvorrichtung eingebracht und die Bestrahlung der Formkörper 16 mit Gamma- oder Röntgenstrahlung durchgeführt, wobei während der Bestrahlung vorzugsweise die Zieltemperatur der Formkörper 16 durch das Heizmittel 18 mit Hilfe der Temperatursensoren 22 und des Steuermittels gehalten wird. Nach dem Ende der Bestrahlung werden die Formkörper 16 in dem Aufnahmeraum 14 abkühlen gelassen, wobei die Abkühlgeschwindigkeit durch das Heizmittel 18 beeinflusst werden kann.

Ein exemplarischer Temperaturverlauf bei der Durchführung des erfindungsgemäßen Verfahrens ist in dem Diagramm der Figur 4 dargestellt. In diesem Beispiel werden die Formkörper zunächst bis auf eine Zieltemperatur von ca. 140 °C erwärmt (Vorheizen), wobei diese Zieltemperatur etwas über der Schmelztemperatur des UHMWPE von ca. 137 °C liegt. Diese Zieltemperatur der Formkörper wird während des Zeitraums der Bestrahlung von 3 bis 4 Stunden gehalten. Nach Abschluss der Bestrahlung erfolgt ein Abkühlen der Formkörper bis auf die Umgebungstemperatur.

Das erfindungsgemäße Verfahren ermöglicht somit einen stetigen und reproduzierbaren Temperaturverlauf der Formkörper, wodurch die aus der Bestrahlung resultierende Vernetzung des UHMWPE effizient und reproduzierbar durchgeführt werden kann. Insbesondere wird bei dem erfindungsgemäßen Verfahren ein zwischenzeitliches Abkühlen der Formkörper nach dem Vorheizen und vor dem Bestrahlen verhindert, wie es bei den Verfahren gemäß dem Stand der Technik typischerweise auftritt.

### Bezugszeichenliste

- 10: Aufnahmevorrichtung
- 12: Wandung
- 14: Aufnahmeraum
- 16: Formkörper aus UHMWPE
- 18: Heizmittel
- 20: Akkumulator
- 22: Temperatursensoren
- 30: Aufnahmevorrichtung
- 40: Aufnahmevorrichtung

## Patentansprüche

1. Verfahren zur Herstellung von einem oder mehreren Formkörpern (16) auf Basis von vernetztem UHMWPE, umfassend die Schritte:
- Verdichten von UHMWPE in Pulverform zu einem oder mehreren Formkörpern (16);
- Einbringen des oder der Formkörper (16) in einen Aufnahmeraum (14) einer Aufnahmevorrichtung (10; 30; 40), wobei die Aufnahmevorrichtung (10; 30; 40) ein Heizmittel (18) zum Erwärmen des oder der in dem Aufnahmeraum befindlichen Formkörper (16) umfasst;
- Bestrahlen des oder der Formkörper (16) in der Aufnahmevorrichtung (10; 30; 40) mit Gamma- oder Röntgenstrahlung, um das UHMWPE zu vernetzen; und
- Entnehmen des oder der Formkörper (16) aus dem Aufnahmeraum (14),
wobei die Aufnahmevorrichtung (10; 30; 40) dafür ausgelegt ist, einen vorgegebenen Temperaturverlauf des oder der Formkörper (16) vor, während und nach dem Bestrahlen zu steuern.

2. Verfahren nach Anspruch 1, wobei der oder die Formkörper (16) nach dem Einbringen in den Aufnahmeraum (14) und vor dem Bestrahlen durch das Heizmittel (18) auf eine Zieltemperatur erwärmt werden.

3. Verfahren nach Anspruch 2, wobei die Zieltemperatur im Bereich der Schmelztemperatur des UHMWPE oder darüber liegt, wobei die Zieltemperatur bevorzugt von 135 bis 150 °C beträgt, weiter bevorzugt von 137 bis 145 °C.

4. Verfahren nach Anspruch 2 oder 3, wobei der oder die Formkörper (16) während dem Bestrahlen im Wesentlichen konstant bei der Zieltemperatur gehalten werden; oder wobei die Temperatur des oder der Formkörper (16) während dem Bestrahlen geändert wird.

5. Verfahren nach einem der vorhergehenden Ansprüche, wobei die Aufnahmevorrichtung (10; 30; 40) ein Steuermittel und einen oder mehrere Temperatursensoren (22) zum Regeln des Temperaturverlaufs des oder der Formkörper (16) vor, während und nach dem Bestrahlen umfasst.

6. Verfahren nach einem der vorhergehenden Ansprüche, wobei das Heizmittel (18) ein Heißluftgebläse, einen Infrarotstrahler oder ein elektrisches Heizelement umfasst.

7. Verfahren nach einem der vorhergehenden Ansprüche, wobei das Bestrahlen des oder der Formkörper (16) mit einer Strahlendosis von 25 bis 45 kGy durchgeführt wird, bevorzugt von 27 bis 33 kGy, weiter bevorzugt von ca. 30 kGy.

8. Verfahren nach einem der vorhergehenden Ansprüche, wobei das Bestrahlen mit Gamma- oder Röntgenstrahlung über einen Zeitraum von 2 bis 6 h durchgeführt wird, bevorzugt von 3 bis 4 h.

9. Verfahren nach einem der vorhergehenden Ansprüche, wobei das UHMWPE in Pulverform vor dem Verdichten mit einem Antioxidans versetzt wird, welches bevorzugt ausgewählt ist aus Tocopherolen, Tocotrienolen, Ascorbinsäure, polyphenolischen Antioxidantien wie z.B. Flavonoiden, Butylhydroxytoluol und Butylhydroxyanisol, und wobei das Antioxidans weiter bevorzugt α-Tocopherol ist.

10. Verfahren nach Anspruch 9, wobei das UHMWPE mit 0,05 bis 0,15 Gew.% des Antioxidans versetzt wird, bevorzugt von 0,09 bis 0,11 Gew.%.

11. Formkörper (16) auf Basis von vernetztem UHMWPE, der gemäß dem Verfahren nach einem der vorhergehenden Ansprüche hergestellt ist.

12. Formkörper (16) nach Anspruch 11, wobei der Formkörper (16) ein Gleitflächenelement für eine Gelenkendoprothese ist, insbesondere für eine Hüftgelenkendoprothese oder eine Kniegelenkendoprothese.

13. System zur Herstellung von einem oder mehreren Formkörpern (16) auf Basis von vernetztem UHMWPE mit einer Aufnahmevorrichtung (10; 30; 40), umfassend einen Aufnahmeraum (14) zur Aufnahme von einem oder mehreren Formkörpern (16) aus verdichtetem UHMWPE und ein Heizmittel (18) zum Erwärmen des oder der in dem Aufnahmeraum (14) befindlichen Formkörper (16), und mit einer Bestrahlungsvorrichtung zum Bestrahlen des oder der Formkörper (16) in der Aufnahmevorrichtung (10; 30; 40) mit Gamma- oder Röntgenstrahlung, um das UHMWPE zu vernetzen, wobei die Aufnahmevorrichtung (10; 30; 40) dafür ausgelegt ist, einen vorgegebenen Temperaturverlauf des oder der Formkörper (16) vor, während und nach dem Bestrahlen zu steuern.

14. System nach Anspruch 13, wobei die Aufnahmevorrichtung (10; 30; 40) ein Steuermittel und einen oder mehrere Temperatursensoren (22) zum Regeln des Temperaturverlaufs des oder der Formkörper vor, während und nach dem Bestrahlen umfasst.

15. System nach Anspruch 13 oder 14, wobei das Heizmittel (18) ein Heißluftgebläse, einen Infrarotstrahler oder ein elektrisches Heizelement umfasst.

## Claims

1. A method for the manufacture of one or more moulded bodies (16) based on cross-linked UHMWPE, comprising the steps:
• compressing UHMWPE in powder form into one or more moulded bodies (16);
• introducing the moulded body or bodies (16) into a receiving space (14) of a receiving device (10; 30; 40), wherein the receiving device (10; 30; 40) comprises a heating means (18) for heating the moulded body or bodies (16) located in the receiving space;
• irradiating the moulded body or bodies (16) in the receiving device (10; 30; 40) with gamma - or X-rays, in order to cross-link the UHMWPE; and
• removing the moulded body or bodies (16) from the receiving space (14),
wherein the receiving device (10; 30; 40) is set up to control a pre-determined temperature behaviour of the moulded body or bodies (16) before, during and after the irradiation.

2. A method according to claim 1, wherein the moulded body or bodies (16) are heated to a target temperature after the introduction into the receiving space (14) and before the irradiation by the heating means (18).

3. A method according to claim 2, wherein the target temperature lies in the region of or over the melting temperature of the UHMWPE, wherein the target temperature is preferably from 135 to 150°C, further preferably from 137 to 145°C.

4. A method according to claim 2 or 3, wherein the moulded body or bodies (16) are held substantially constantly at the target temperature during the irradiation; or wherein the temperature of the moulded body or bodies (16) is changed during the irradiation.

5. A method according to any one of the preceding claims, wherein the receiving device (10; 30; 40) comprises a control means and one or more temperature sensors (22) for controlling the temperature behaviour of the moulded body or bodies (16) before, during and after the irradiation.

6. A method according to any one of the preceding claims, wherein the heating means (18) comprises a hot air blower, an infrared radiator or an electrical heating element.

7. A method according to any one of the preceding claims, wherein the irradiation of the moulded body or bodies (16) is executed with a radiation dose of 25 to 45kGy, preferably of 27 to 33kGy, further preferably of approximately 30kGy.

8. A method according to any one of the preceding claims, wherein the radiation is executed with gamma - or X-ray radiation over a time period of 2 to 6 hours, preferably of 3 to 4 hours.

9. A method according to any one preceding claims, wherein the UHMWPE in powder form is cross-linked before the compression with an antioxidant, which is preferably selected from tocopherols, tocotrienols, ascorbic acid, polyphenolic antioxidants such as for example flavonoids, butylated hydroxytoluene and butylated hydroxyanisole, and wherein the antioxidant is further preferably α-tocopherol.

10. A method according to claim 9, wherein the UHMWPE is cross-linked with 0.05 to 0.15wt% of the antioxidant, preferably with 0.09 to 0.11wt%.

11. A moulded body (16) based on cross-linked UHMWPE, which is manufactured according to the method according to any one of the preceding claims.

12. A moulded body (16) according to claim 11, wherein the moulded body (16) is a sliding surface element for a joint endoprosthesis, in particular for a hip joint endoprosthesis or a knee joint endoprosthesis.

13. A system for the manufacture of one or more moulded bodies (16) based on cross-linked UHMWPE with a receiving device (10; 30; 40), comprising a receiving space (14) for receiving one or more moulded bodies (16) of cross-linked UHMWPE and a heating means (18) for heating the moulded body or bodies (16) located in the receiving space (14), and with an irradiation device for irradiating the moulded body or bodies (16) in the receiving device (10; 30; 40) with gamma - or X-ray radiation, in order to cross-link the UHMWPE, wherein the receiving device (10; 30; 40) is set up to control a predetermined temperature behaviour of the moulded body or bodies (16) before, during and after the irradiation.

14. A system according to claim 13, wherein the receiving device (10; 30; 40) comprises a control means and one or more temperature sensors (22) for controlling the temperature behaviour of the moulded body or bodies (16) before, during and after the irradiation.

15. A system according to claim 13 or 14, wherein the heating means (18) comprises a heating blower, an infrared radiator or an electrical heating element.

## Revendications

1. Procédé de fabrication d'un ou plusieurs corps moulés (16) à base d'UHMWPE réticulé, comprenant les étapes suivantes :
- compactage d'UHMWPE sous forme de poudre en un ou plusieurs corps moulés (16) ;
- introduction du ou des corps moulés (16) dans un espace de réception (14) d'un dispositif de réception (10 ; 30 ; 40), le dispositif de réception (10 ; 30 ; 40) comprenant un moyen de chauffage (18) pour chauffer le ou les corps moulés (16) se trouvant dans l'espace de réception (14) ;
- irradiation du ou des corps moulés (16) dans le dispositif de réception (10 ; 30 ; 40) avec un rayonnement gamma ou X, afin de réticuler l'UHMWPE ; et
- retrait du ou des corps moulés (16) de l'espace de réception (14),
le dispositif de réception (10 ; 30 ; 40) étant conçu pour contrôler un profil de température prédéfini du ou des corps moulés (16) avant, pendant et après l'irradiation.

2. Procédé selon la revendication 1, dans lequel le ou les corps moulés (16) sont chauffés à une température cible par le moyen de chauffage (18) après leur introduction dans l'espace de réception (14) et avant l'irradiation.

3. Procédé selon la revendication 2, dans lequel la température cible est de l'ordre de la température de fusion de l'UHMWPE ou supérieure, la température cible étant de préférence de 135 à 150 °C, plus préférablement de 137 à 145 °C.

4. Procédé selon la revendication 2 ou 3, dans lequel le ou les corps moulés (16) sont maintenus à la température cible de manière sensiblement constante pendant l'irradiation ; ou dans lequel la température du ou des corps moulés (16) est modifiée pendant l'irradiation.

5. Procédé selon l'une des revendications précédentes, dans lequel le dispositif de réception (10 ; 30 ; 40) comprend un moyen de commande et un ou plusieurs capteurs de température (22) pour réguler le profil de température du ou des corps moulés (16) avant, pendant et après l'irradiation.

6. Procédé selon l'une des revendications précédentes, dans lequel le moyen de chauffage (18) comprend un souffleur à air chaud, un radiateur infrarouge ou un élément chauffant électrique.

7. Procédé selon l'une des revendications précédentes, dans lequel l'irradiation du ou des corps moulés (16) est effectuée avec une dose de rayonnement de 25 à 45 kGy, de préférence de 27 à 33 kGy, plus préférablement d'environ 30 kGy.

8. Procédé selon l'une des revendications précédentes, dans lequel l'irradiation avec un rayonnement gamma ou X est effectuée sur une période de 2 à 6 heures, de préférence de 3 à 4 heures.

9. Procédé selon l'une des revendications précédentes, dans lequel, à l'UHMWPE sous forme de poudre, est ajouté un antioxydant avant le compactage, lequel est de préférence choisi parmi les tocophérols, les tocotriénols, l'acide ascorbique, les antioxydants polyphénoliques tels que les flavonoïdes, le butylhydroxytoluène et le butylhydroxyanisole, et l'antioxydant est plus préférablement l'α-tocophérol.

10. Procédé selon la revendication 9, dans lequel l'UHMWPE est additionné de 0,05 à 0,15 % en poids de l'antioxydant, de préférence de 0,09 à 0,11 % en poids.

11. Corps moulé (16) à base d'UHMWPE réticulé, fabriqué selon le procédé selon l'une des revendications précédentes.

12. Corps moulé (16) selon la revendication 11, dans lequel le corps moulé (16) est un élément de surface de glissement pour une endoprothèse articulaire, en particulier pour une endoprothèse de hanche ou une endoprothèse de genou.

13. Système de fabrication d'un ou plusieurs corps moulés (16) à base d'UHMWPE réticulé, comprenant un dispositif de réception (10 ; 30 ; 40), comprenant un espace de réception (14) pour loger un ou plusieurs corps moulés (16) en UHMWPE compacté et un moyen de chauffage (18) pour chauffer le ou les corps moulés (16) se trouvant dans l'espace de réception (14), et avec un dispositif d'irradiation pour irradier le ou les corps moulés (16) dans le dispositif de réception (10 ; 30 ; 40) avec un rayonnement gamma ou X, afin de réticuler l'UHMWPE, le dispositif de réception (10 ; 30 ; 40) étant conçu pour contrôler un profil de température prédéfini du ou des corps moulés (16) avant, pendant et après l'irradiation.

14. Système selon la revendication 13, dans lequel le dispositif de réception (10 ; 30 ; 40) comprend un moyen de commande et un ou plusieurs capteurs de température (22) pour réguler le profil de température du ou des corps moulés avant, pendant et après l'irradiation.

15. Système selon la revendication 13 ou 14, dans lequel le moyen de chauffage (18) comprend un souffleur à air chaud, un radiateur infrarouge ou un élément chauffant électrique.
